# EUROPEAN PATENT APPLICATION

(11) **EP 4 226 974 A1**
(43) Date of publication of application: **16.08.2023**
(21) Application number: 21877486.7
(22) Date of filing: 30.09.2021
(51) Int. Cl.: A61P 39/06, A61P 43/00, A61P 17/16, A61P 17/18, A61Q 17/04, A61Q 19/00, A61Q 19/02, A61K 36/28, A23L 19/00, A23L 33/105, A61K 8/49, A61K 8/9789, A61K 31/352

(54) **METHOD FOR PRODUCING EXTRACT COMPOSITION**

(30) Priority: 05.10.2020 JP 2020168623
(71) Applicant: Kao Corporation, Chuo-ku Tokyo 103-8210 (JP)
(72) Inventor: DOI Haruhiko, Wakayama-shi, Wakayama 640-8580 (JP); KAYAKUBO Daisuke, Wakayama-shi, Wakayama 640-8580 (JP); YAMAZAWA Yuka, Wakayama-shi, Wakayama 640-8580 (JP)
(74) Representative: Hoffmann Eitle
(86) International application number: PCT/JP2021/036197
(87) International publication number: WO 2022/075185

(57) **Abstract**

In an aspect, the present disclosure provides a method for producing an extract composition by which polyphenols such as apigenin and/or essential oil components such as spiro-ether can be efficiently extracted from harvested herbaceous plants.

An aspect of the present disclosure is directed to a method for producing an extract composition, including processes (1) and (2) below:
(1) applying an oxidative stress to a harvested herbaceous plant and preserving it; and
(2) obtaining an extract from the herbaceous plant that has undergone the process (1).

## Description

### Technical Field

The present disclosure relates to a method for producing an extract composition, a method for producing an external product or food, and an extract composition and an external product or food.

### Background Art

Apigenin, which is a flavonoid (a type of polyphenol) found in plants such as German chamomile (Matricaria recutita L.), Roman chamomile (Anthemis nobilis L.), dahlia (Dahlia pinnata), lilac daphne (Daphne genkwa), and kaoliang (Sorghum nervosum Bess), is known to have urease inhibitory properties, antioxidative properties, melanogenesis-stimulating properties and other properties, and be useful as an ingredient of external products including cosmetics, pharmaceuticals, and quasi-drugs.

Plants containing flavonoids such as apigenin are typically grown in plant factories, and various methods for growing flavonoid-containing plants and extracting flavonoid-containing extracts have been studied.

For example, Patent Publication No. 5592620 (Patent Document 1) proposes a plant growing method by which the amount of polyphenols in plants is increased by applying an oxidative stress to plants to grow.

JP 2010-163363 A (Patent Document 2) proposes an extraction method for obtaining extracts having a high concentration of apigenin by causing extracted substances from apigenin-containing plants to adsorb on a carrier, washing it with water or an ethanol aqueous solution having a concentration of 20% by volume or less, and eluting apigenin from the adsorbed substances using an ethanol aqueous solution having a concentration of 40 to 99.5% by volume.

WO 2018/151334 A1 (Patent Document 3) teaches that chamomiles such as German chamomile and Roman chamomile include not only polyphenol components such as apigenin, quercetin, patuletin, and luteolin, but also essential oil components such as bisabolol, spiro-ether, azulenes (e.g., chamazulene), and oxides thereof. JP 2018-193323 A (Patent Document 4) teaches that spiro-ether has a skin -lightening effect and is used as an active ingredient of emulsion cosmetics.

### Summary of Invention

An aspect of the present disclosure is directed to a method for producing an extract composition, including processes (1) and (2) below:
(1) applying an oxidative stress to a harvested herbaceous plant and preserving it; and
(2) obtaining an extract from the herbaceous plant that has undergone the process (1).

An aspect of the present disclosure is directed to a method for producing an extract composition, including processes (1) and (2) below:
(1) applying an oxidative stress to a harvested apigenin-containing plant and preserving it; and
(2) obtaining an extract from the apigenin-containing plant that has undergone the process (1).

An aspect of the present disclosure is directed to a method for producing an external product or food, including a process of producing an extract composition by the method for producing an extract composition of the present disclosure.

An aspect of the present disclosure is directed to an extract composition that is produced by the method for producing an extract composition of the present disclosure, wherein the extract composition contains apigenin in an amount of 0.001% by mass or more.

An aspect of the present disclosure is directed to an external product or food containing the extract composition of the present disclosure or a purified product thereof.

### Description of the Invention

As described above, plants containing polyphenols such as apigenin and plants containing essential oil components such as spiro-ether are typically grown in plant factories, which is costly. More efficient methods for extracting polyphenols such as apigenin or essential oil components such as spiro-ether from herbaceous plants have been demanded.

In view of the above, the present disclosure provides a method for producing an extract composition by which polyphenols such as apigenin and/or essential oil components such as spiro-ether can be efficiently extracted from harvested herbaceous plants containing polyphenols such as apigenin and/or essential oil components such as spiro-ether, a method for producing an external product or food using the same, and an extract composition and an external product or food.

The present disclosure is based on the finding that applying an oxidative stress to a harvested herbaceous plant containing polyphenols such as apigenin and/or essential oil components such as spiro-ether and preserving it can increase the amount of polyphenols such as apigenin and/or essential oil components such as spiro-ether in an extract as compared with the case of not applying an oxidative stress, and thus more efficiently extracting polyphenols such as apigenin and/or essential oil components such as spiro-ether from the harvested herbaceous plant containing polyphenols such as apigenin and/or essential oil components such as spiro-ether.

An aspect of the present disclosure is directed to a method for producing an extract composition, including processes (1) and (2) below (hereinafter also referred to as a "production method of the extract composition of the present disclosure"):
(1) applying an oxidative stress to a harvested herbaceous plant and preserving it; and
(2) obtaining an extract from the herbaceous plant that has undergone the process (1).

In one or more embodiments, the production method of the extract composition of the present disclosure is a method for producing an extract composition, including processes (1) and (2) below:
(1) applying an oxidative stress to a harvested apigenin-containing plant and preserving it; and
(2) obtaining an extract from the apigenin-containing plant that has undergone the process (1).

The detailed mechanism for expressing such effects of the present disclosure is unclear, but the following is considered.

Polyphenols such as apigenin are a kind of antioxidative substances. When an oxidative stress is externally applied to harvested herbaceous plants containing polyphenols such as apigenin, they produce polyphenols such as apigenin inside their bodies to antagonize the oxidative stress. Further, it is considered that preservation after application of an oxidative stress can induce the production of polyphenols such as apigenin.

The mechanism is unclear also as to the surprising effect that, when an oxidative stress is externally applied to herbaceous plants containing essential oil components such as spiro-ether, the production of essential oil components such as spiro-ether is induced during preservation after application of an oxidative stress.

The present disclosure, however, is not limited to these mechanisms.

In an aspect, the present disclosure provides a method for producing an extract composition by which polyphenols such as apigenin and/or essential oil components such as spiro-ether can be efficiently extracted from harvested herbaceous plants containing polyphenols such as apigenin and/or essential oil components such as spiro-ether.

In one or more embodiments, the present disclosure can increase the proportion of polyphenols such as apigenin and/or essential oil components such as spiro-ether in extracts, thereby enabling polyphenols such as apigenin and/or essential oil components such as spiro-ether to be extracted efficiently. In one or more embodiments, the present disclosure provides extracts with high proportions of polyphenols such as apigenin and/or essential oil components such as spiro-ether. External products such as cosmetics or food can be produced using such extracts.

In one or more embodiments of the present disclosure, the term "polyphenols" refer to compounds having two or more phenolic hydroxyl groups in the same molecule (hydroxyl groups bonded to aromatic rings such as benzene and naphthalene rings). In one or more embodiments, examples of the polyphenols include flavonoid compounds such as flavones.

In one or more embodiments of the present disclosure, examples of the polyphenols include apigenin, rutin, luteolin, chlorogenic acid, and rosmarinic acid.

In one or more embodiments of the present disclosure, examples of the essential oil components include chamazulene, umbelliferone, 7-methoxycoumarin, matricin, matricarin, taraxasterol, lupeol, apiin, and spiro-ether.

In one or more embodiments of the present disclosure, examples of the herbaceous plant include herbaceous plants containing polyphenols and/or essential oil components. In another one or more embodiments, examples thereof include herbaceous plants containing extraction target components.

Any herbaceous plant that contains polyphenols such as apigenin and/or essential oil components such as spiro-ether may be used as the herbaceous plant of the present disclosure. In one or more embodiments, examples of the herbaceous plant include plants containing apigenin, plants containing spiro-ether, and plants containing apigenin and spiro-ether. From the viewpoint of an abundance of plant flavonoids, examples thereof include Asteraceae, Umbelliferae, Lamiaceae, and Guttiferae plants. As the Asteraceae plants, German chamomile (Japanese name: kamitsure), Roman chamomile (Japanese name: Roman kamitsure) and their relative species are preferable from the viewpoint of efficiently extracting apigenin and/or spiro-ether, and Roman chamomile is more preferable from the viewpoint of efficiently extracting apigenin. The Umbelliferae plant is, for example, Angelica keiskei. The Lamiaceae plant is, for example, peppermint. The Guttiferae plant is, for example, Saint John's wort.

### [Process (1)]

The process (1) in the production method of the extract composition of the present disclosure is a process of applying an oxidative stress to a harvested herbaceous plant and preserving it. In the present disclosure, the term "preservation" encompasses still standing, still standing without attention, and storage.

In one or more embodiments, the harvested herbaceous plant in the process (1) is a part or an entire of the herbaceous plant. The site of the plant to apply an oxidative stress can be determined appropriately depending on the type of the plant. The part of the herbaceous plant may be, for example, the aerial parts of the plant including flowers, petals, buds, leaves, and stems. For example, when German chamomile or Roman chamomile is used as the herbaceous plant, the part of the herbaceous plant is preferably flowers, petals, or buds, and more preferably petals, from the viewpoint of inducing the production of apigenin in response to an oxidative stress and from the viewpoint of efficiently extracting apigenin.

The herbaceous plant in the process (1) is preferably those within seven days of harvest, more preferably those within three days of harvest, and further preferably those within one day of harvest, from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether. The herbaceous plant in the process (1) is preferably those after a lapse of at least 30 minutes of harvest, more preferably those after a lapse of at least one hour of harvest, and further preferably those after a lapse of at least 12 hours of harvest, from the same viewpoints.

In one or more embodiments, the application of an oxidative stress in the process (1) is preferably an application of an oxidative stress with use of a liquid composition containing an oxidizing agent, from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether. The term "liquid composition" in the present disclosure refers to a liquid composition for applying an oxidative stress, unless otherwise specified. The liquid composition may be simply an oxidizing agent or an oxidizing agent aqueous solution. The oxidizing agent, etc., will be described later.

In one or more embodiments, the method for applying an oxidative stress with use of the liquid composition may be a method of bringing the liquid composition into contact with the harvested herbaceous plant. The exemplary methods of bringing the liquid composition into contact with the harvested herbaceous plant include a method of dropping the liquid composition on the harvested herbaceous plant, a method of spraying the liquid composition onto the harvested herbaceous plant, and a method of immersing the harvested herbaceous plant in the liquid composition.

In the case of immersing the harvested herbaceous plant in the liquid composition, the immersion time is preferably 6 hours or more, more preferably 12 hours or more, further preferably 24 hours or more, and preferably 240 hours or less, more preferably 200 hours or less, and further preferably 170 hours or less, from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether. In the case of immersing the herbaceous plant in the liquid composition, the preservation after the application of an oxidative stress in the process (1) may be preservation in a state where the herbaceous plant is immersed in the liquid composition or preservation after the herbaceous plant is taken out from the liquid composition.

In one or more embodiments of the present disclosure, the method for applying an oxidative stress does not include irradiation with ultraviolet light.

The amount of the liquid composition to be used for the application of an oxidative stress is not particularly limited as long as the liquid composition is sufficiently brought into contact with the herbaceous plant. For example, the amount of the liquid composition to be brought into contact with the herbaceous plant is preferably 1.5 times or more, more preferably 2 times or more, and further preferably 2.5 times or more relative to the wet weight of the herbaceous plant from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether, whereas the amount thereof is preferably 5000 times or less, more preferably 1000 times or less, and further preferably 100 times or less relative to the wet weight of the herbaceous plant from the viewpoint of the cost reduction. The term "wet weight" in the present disclosure refers to the weight of the herbaceous plant after harvest, the weight of the herbaceous plant that has not been completely dried, the weight of the herbaceous plant that has not been subjected to a drying treatment after harvest, or the weight of the herbaceous plant with moisture.

In the process (1), in the case of not using cold preservation or freezing preservation, the preservation period of the herbaceous plant after the application of an oxidative stress is preferably 12 hours or more, more preferably 24 hours or more, and further preferably 48 hours or more from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether, whereas the preservation period is preferably 240 hours or less, more preferably 200 hours or less, and further preferably 170 hours or less from the same viewpoints. The preservation period in the case of using cold preservation or freezing preservation is preferably 12 hours or more, more preferably 24 hours or more, and further preferably 48 hours or more from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether, whereas the preservation period is preferably 120 days or less, more preferably 150 days or less, and further preferably 180 days or less from the same viewpoints.

In the process (1) in one or more embodiments, an oxidative stress may be further applied during the preservation period of the herbaceous plant after the application of an oxidative stress. In the case of further applying an oxidative stress during the preservation period, the number of times of the application of an oxidative stress during the preservation period may be one time, or two or more times. In the case of applying an oxidative stress a plurality of times during the preservation period, the application of an oxidative stress may be performed at regular or irregular intervals during the preservation period. The term "preservation period" in the present disclosure refers to a period between the initial application of an oxidative stress and the start of the process (2).

In the process (1) in one or more embodiments, the herbaceous plant may be subjected to a drying treatment during the preservation period of the herbaceous plant after the application of an oxidative stress. The drying treatment method may be drying in the sun, drying in the shade, drying using a dryer, or the like.

In the process (1), the herbaceous plant may be irradiated with ultraviolet light during the preservation period of the herbaceous plant after the application of an oxidative stress from the viewpoint of inducing the production of polyphenols such as apigenin and/or essential oil components such as spiro-ether in response to an oxidative stress and from the viewpoint of more efficiently extracting polyphenols such as apigenin and/or essential oil components such as spiro-ether. The ultraviolet light irradiation method may be irradiation with UV light, drying in the sun, or the like.

In the process (1), the temperature at the time of applying an oxidative stress and the temperature during the preservation of the herbaceous plant after the application of an oxidative stress are preferably 10°C or more, more preferably 15°C or more, and further preferably 20°C or more from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether, whereas the temperatures are preferably 40°C or less, more preferably 35°C or less, and further preferably 30°C or less from the same viewpoints.

In the process (1), the humidity at the time of applying an oxidative stress and the humidity during the preservation of the herbaceous plant after the application of an oxidative stress are preferably 40% or more, more preferably 50% or more, and further preferably 60% or more from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether, whereas the humidities are preferably 90% or less, more preferably 80% or less, and further preferably 70% or less from the same viewpoints.

### (Liquid Composition for Applying Oxidative Stress)

In one or more embodiments, the liquid composition for applying an oxidative stress in the process (1) contains an oxidizing agent and an aqueous medium. Examples of the oxidizing agent to be contained in the liquid composition include hydrogen peroxide, hypochlorous acid, nitric acid, potassium nitrate, permanganate, peroxide, ozone, and sulfur dioxide. The oxidizing agent may be one kind or a combination of two or more kinds.

The content of the oxidizing agent in the liquid composition is preferably 0.0001 mM or more, more preferably 0.01 mM or more, and further preferably 0.1 mM or more from the viewpoint of inducing the production of polyphenols such as apigenin and/or essential oil components such as spiro-ether in response to an oxidative stress and from the viewpoint of efficiently extracting polyphenols such as apigenin and/or essential oil components such as spiro-ether, whereas the content thereof is preferably 5 mM or less, more preferably 1 mM or less, and further preferably 0.5 mM or less from the same viewpoints. When the oxidizing agent is a combination of two or more kinds, the content of the oxidizing agent in the liquid composition is the total content of the two or more kinds.

In one or more embodiments of the present disclosure, the aqueous medium to be contained in the liquid composition is water.

The content of the aqueous medium or water in the liquid composition may be a remainder after subtracting the oxidizing agent and a spreading agent and other components, which are described below, from the total amount of the liquid composition.

The liquid composition may contain a spreading agent in addition to the oxidizing agent. In one or more embodiments, the spreading agent is a functional spreading agent (adjuvant). In one or more embodiments, the functional spreading agent has a function of promoting penetration and transfer of chemical solution components into plants. Examples of the functional spreading agent include surfactants such as nonionic surfactants, anionic surfactants, and cationic surfactants. Among these, nonionic surfactants are preferable from the viewpoint of inducing the production of apigenin in response to an oxidative stress and from the viewpoint of efficiently extracting apigenin. Specific examples of the functional spreading agent include polyoxyethylene hexitane fatty acid ester (the average number of added moles of ethylene group, 1 to 150), polyoxyalkylene such as polyoxyethylene sorbitan monooleate (the average number of added moles of ethylene group, 1 to 150), polyoxyethylene nonylphenyl ether (the average number of added moles of ethylene group, 1 to 150), polyoxyethylene alkyl ether (the average number of added moles of ethylene group, 1 to 150, and the number of carbon atoms in alkyl group, 4 to 22), polyalkylene glycol alkyl ether (the average number of added moles of ethylene group, 1 to 150, and the number of carbon atoms in alkyl group, 4 to 22), and polyoxyethylene fatty acid ester (the average number of added moles of ethylene group, 1 to 150). Among these, polyoxyethylene sorbitan monooleate is preferable. Examples of commercially available spreading agents include "Approach BI" (produced by MARUWABiochemical Co., Ltd.) and "RHEODOL TW-0120V" (manufactured by Kao Corporation). The spreading agent may be one kind or a combination of two or more kinds.

When the liquid composition contains the spreading agent, the content of the spreading agent in the liquid composition is preferably 0.001% by mass or more, more preferably 0.01% by mass or more, and further preferably 0.1% by mass or more from the viewpoint of inducing the production of apigenin in response to an oxidative stress and from the viewpoint of efficiently extracting apigenin, whereas the content thereof is preferably 5% by mass or less, more preferably 1% by mass or less, and further preferably 0.3% by mass or less from the same viewpoints. When the spreading agent is a combination of two or more kinds, the content of the spreading agent in the liquid composition is the total content of the two or more kinds.

In one or more embodiments, the liquid composition may contain other components as appropriate. Examples of the other components include a moisturizing agent for preventing drying and an antiseptic agent for preventing decay.

In one or more embodiments, the process (1) may include a process of drying the herbaceous plant (drying process) after application of an oxidative stress and preservation of the herbaceous plant, from the viewpoint of efficiently extracting polyphenols such as apigenin and/or essential oil components such as spiro-ether.

The drying time in the drying process is preferably 12 hours or more, more preferably 24 hours or more, and further preferably 36 hours or more from the same viewpoint.

The drying treatment method in the drying process is not particularly limited as long as it can dry the herbaceous plant. For example, a drying method using a thermostat at 40°C may be used.

As a criterion for determining the dry state of the herbaceous plant, for example, if the change in weight of the plant is 3% or less in the period of 24 hours in the drying process, the plant may be judged as being in a dry state.

### [Process (2)]

The process (2) in the production method of the extract composition of the present disclosure is a process of obtaining an extract from the herbaceous plant that has undergone the process (1). The extraction method of an extract may be a method of bringing the herbaceous plant that has undergone the process (1) into contact with an extraction solvent to elute an extract from the herbaceous plant. The method of bringing the herbaceous plant into contact with the extraction solvent may be a method of immersing the herbaceous plant in the extraction solvent. In one or more embodiments, the extract may be obtained through filtration with a filter of the extraction solvent that has been used for immersion of the herbaceous plant. In one or more embodiments, the process (2) is a process of obtaining an extract by immersing the herbaceous plant that has undergone the process (1) in an elution solvent and filtering the solvent with a filter.

Examples of the extraction target components in the extract composition according to the present disclosure, i.e., components of the extract composition obtained in the process (2), include polyphenols such as apigenin and essential oil components such as spiro-ether.

The extraction solvent for polyphenols such as apigenin may be either a polar or non-polar solvent. Examples of the extraction solvent include water; alcohols such as methanol, ethanol, propanol, and butanol; polyhydric alcohols such as propylene glycol and butylene glycol; ketones such as acetone and methyl ethyl ketone; esters such as methyl acetate and ethyl acetate; chain and cyclic ethers such as tetrahydrofuran and diethyl ether; polyethers such as polyethylene glycol; hydrocarbons such as squalane, hexane, cyclohexane, petroleum ether; aromatic hydrocarbons such as toluene; halogenated hydrocarbons such as dichloromethane, chloroform, and dichloroethane; and carbon dioxides. Among these, a butylene glycol aqueous solution is preferable from the viewpoint of efficiently extracting polyphenols such as apigenin. The concentration of the butylene glycol aqueous solution is preferably 20% by mass to 90% by mass.

The extraction solvent for essential oil components such as spiro-ether is not particularly limited as long as it is oil-soluble, but it is preferably a lipophilic organic solvent. The lipophilic organic solvent is preferably an oil solution having a solubility parameter (SP value) ranging from 15 to 21, and examples thereof include isopropyl myristate (SP value, 17.0), neopentyl glycol dicaprate (SP value, 17.7), liquid paraffin (SP value, 16.4), squalane (SP value, 16.2), and mixed solvents of two or more of these. These may be oils of plant origin such as castor oil, persic oil, soybean oil, and sunflower oil. The SP value, which is a measure of compatibility between substances, can be determined by calculating three-dimensional Hansen solubility parameters based on a method described in JP H10-194920 A.

The amount of the extraction solvent for polyphenols such as apigenin relative to the weight or dry weight of the herbaceous plant that has undergone the process (1) is preferably 3 times or more, more preferably 5 times or more, and further preferably 10 times or more from the viewpoint of efficiently extracting polyphenols such as apigenin, and the amount is preferably 100 times or less, more preferably 60 times or less, and further preferably 40 times or less from the same viewpoint.

The time during which flowers and/or leaves are in contact with the extraction solvent for polyphenols such as apigenin or the immersion time during which flowers and/or leaves are immersed in the extraction solvent is preferably 12 hours or more, more preferably 24 hours or more, and further preferably 48 hours or more from the viewpoint of efficiently extracting polyphenols such as apigenin, and the time is preferably 240 hours or less, more preferably 200 hours or less, and further preferably 160 hours or less from the same viewpoint.

The temperature of the extraction solvent for bringing flowers and/or leaves into contact with the extraction solvent or the immersion temperature for immersing flowers and/or leaves in the extraction solvent is preferably 5°C or more, more preferably 10°C or more, and further preferably 15°C or more from the viewpoint of efficiently extracting polyphenols such as apigenin, and the temperature is preferably 60°C or less, more preferably 50°C or less, and further preferably 40°C or less from the same viewpoint.

The filter to be used for filtration is, for example, filter paper, a membrane filter, a cartridge filter, a disposable filter, or the like.

The essential oil components such as spiro-ether can be extracted by a method described in JP H10-194920 A for example, which discloses extraction from a herbaceous plant with use of a lipophilic organic solvent. In one or more embodiments, the essential oil components such as spiro-ether may be extracted by adding, to pulverized dry herbaceous plant, a lipophilic organic solvent in an amount of 1 to 100 times by mass relative to the amount of flowers and/or leaves and subjecting it to extraction under stirring at 10°C to 90°C for 1 to 96 hours.

The extract composition that is produced by the production method of the extract composition of the present disclosure may be purified so as to obtain a purified product thereof. Therefore, in one or more embodiments, the process (2) may include a process of purifying the extract. In another one or more embodiments, the production method of the extract composition of the present disclosure may further include a process of purifying the extract after the process (2).

### [Extract Composition]

The present disclosure in an aspect relates to an extract composition that is produced by the production method of the extract composition of the present disclosure (hereinafter also referred to as an "extract composition of the present disclosure"). In one or more embodiments, the extract composition of the present disclosure contains apigenin in an amount of 0.001% by mass or more. According to the present disclosure, the production method of the extract composition of the present disclosure can efficiently extract polyphenols such as apigenin and/or essential oil components such as spiro-ether from harvested herbaceous plants to produce an extract composition. The amount of polyphenols such as apigenin in the solid content in the extract composition of the present disclosure is preferably 0.005% by mass or more, more preferably 0.01% by mass or more, and further preferably 0.05% by mass or more.

The amount of polyphenols such as apigenin in the extract composition relative to 1 g of the dry weight of the herbaceous plant is preferably 0.01% by mass or more, more preferably 0.03% by mass or more, and further preferably 0.05% by mass or more, and preferably 1% by mass or less, more preferably 0.3% by mass or less, and further preferably 0.1% by mass or less.

The extract composition or a purified product thereof that is produced by the production method of the extract composition of the present disclosure is suitably used as an external products or food. Therefore, an aspect of the present disclosure is directed to a method for producing an external product or food, including a process of producing an extract composition by the production method of the extract composition of the present disclosure. Another aspect of the present disclosure is directed to an external product or food containing the extract composition of the present disclosure or a purified product thereof.

In the present disclosure, examples of the external product include perfumed products such as cosmetics, hair care products, pharmaceuticals, quasi-drugs, bath salts, and toothpastes. The external product may be in the form of a cream, a liquid lotion, an emulsion lotion, a spray, a skin lotion, or the like.

In one or more embodiments, the oxidative stress in the process (1) of the present disclosure is not applied to the extract composition obtained in the process (2).

### Examples

Hereinafter, the present disclosure will be described in more detail by way of examples. However, the following examples are merely illustrative and are not intended to limit the present disclosure.

### 1-1. Preparation of Liquid Compositions for Applying Oxidative Stress

### (Examples 1 to 4)

Oxidizing agent aqueous solutions of Examples 1 to 4 shown in Table 1 were prepared by mixing an oxidizing agent, a spreading agent, and water. Table 1 shows the contents (effective amounts) of the respective components in the oxidizing agent aqueous solutions. The water content in the oxidizing agent aqueous solution is a remainder after subtracting the oxidizing agent and the spreading agent from the total amount of the solution.

The following were used to prepare the oxidizing agent aqueous solutions.

### (Oxidizing Agent)

Hydrogen peroxide solution [manufactured by ADEKA CORPORATION, concentration, 35% by mass]

### (Spreading Agent)

Nonionic surfactant [polyoxyethylene sorbitan monooleate, the average number of added moles of ethylene group, 20, "RHEODOL TW-0120V" manufactured by Kao Corporation, concentration, 51.5% by mass).

### 1-2. Production Method of Extract Composition

### (Examples 1 to 4)

An oxidative stress was applied to harvested chamomile using the prepared oxidizing agent aqueous solutions of Examples 1 to 4 to obtain extract compositions of Examples 1 to 4.

### [Harvest of Asteraceae Plant]

Potted seedlings of Roman chamomile (Japanese name: Roman kamitsure) were planted in a hydroponic culture system (temperature, 25°C) to grow. As a liquid fertilizer for hydroponic culture, HYPONICA (manufactured by KYOWA CO., LTD.) was diluted 1000 times. After flowering of Roman chamomile, only the flowers were harvested.

### [Process (1)]

The oxidizing agent aqueous solution was dropped with a dropper on the flowers within one day of harvest. The amount of the oxidizing agent aqueous solution dropped thereon was three times (2.4 g) the wet weight (unit: 0.8 g) of the flowers. The flowers were blended with the oxidizing agent aqueous solution and left to stand at 25°C for three days under indoor conditions.

### [Process (2)]

The flowers after the process (1) were immersed in a 1,3-butylene glycol aqueous solution (concentration, 60% by mass) in an amount 20 times (3.0 g) the dry weight (0.15 g) of the flowers after the process (1). The immersion temperature was 40°C, and the immersion time was one day.

Thereafter, the 1,3-butylene glycol aqueous solution (immersion solution), in which the flowers were immersed, was filtered with a filter (disposable filter manufactured by ADVANTEC, pore diameter, 0.45 pm) to obtain an extract composition.

### (Comparative Example 1)

An extract composition of Comparative Example 1 was obtained in the same manner as in Examples 1 to 4 except that the oxidizing agent aqueous solution was not dropped on the harvested flowers.

### (Comparative Example 2)

An extract composition of Comparative Example 2 was obtained in the same manner as in Examples 1 to 4 except that the oxidizing agent aqueous solution was dropped on the flowers before harvest.

### 1-3. Evaluation

### [Evaluation of Apigenin Content]

The apigenin content in the obtained extract composition was measured using high performance liquid chromatography (HPLC) under conditions below. Table 1 shows the results of the apigenin concentration, the apigenin concentration relative to the case of not applying an oxidative stress (Comparative Example 1), and the apigenin concentration relative to the case of applying an oxidative stress before harvest (Comparative Example 2).

### <Measurement Conditions>

Column: InertSustain C18 (manufactured by GL Sciences Inc.)
Eluant: formic acid 0.1% (solution A) and acetonitrile (solution B)
Gradient conditions: gradient from 95 (solution A) : 5 (solution B) to 10 (solution A): 90 (solution B)
Flow rate: 0.5 mL/min
Column temperature: 40°C
Detector: CAD

**[Table 1]**

| Table 1 | Liquid composition for applying oxidative stress | | | | Timing to apply oxidative stress to Roman chamomile (flowers) | Extract composition | | |
|---|---|---|---|---|---|---|---|---|
| | Oxidative stress applying agent | | Spreading agent | | | Apigenin concentration (ppm) | Relative to the case of not applying oxidative stress | Relative to the case of applying oxidative stress before harvest |
| | Kind | Concentration | Kind | Concentration (mass%) | | | | |
| Ex. 1 | Hydrogen peroxide | 1 mM | - | - | After harvest | 967 | 1.7 | 1.5 |
| Ex. 2 | Hydrogen peroxide | 0.1 mM | - | - | After harvest | 911 | 1.6 | 1.4 |
| Ex. 3 | Hydrogen peroxide | 0.01 mM | - | - | After harvest | 939 | 1.6 | 1.4 |
| Ex. 4 | Hydrogen peroxide | 0.01 mM | Nonionic surfactant | 0.10 | After harvest | 1048 | 1.8 | 1.6 |
| Comp. Ex. 1 | - | - | - | - | - | 572 | 1.0 | 0.9 |
| Comp. Ex. 2 | Hydrogen peroxide | 0.01 mM | - | - | Before harvest | 665 | 1.2 | 1.0 |

| | | | | | | | | |
|---|---|---|---|---|---|---|---|---|
| * Ex.: Example, Comp. Ex.: Comparative Example | | | | | | | | |

Table 1 above indicates that Examples 1 to 4 in which the application of an oxidative stress with use of the oxidizing agent aqueous solution was performed increased the apigenin concentration in the extract compositions as compared with Comparative Example 1 in which the application of an oxidative stress with use of the oxidizing agent aqueous solution was not performed. Table 1 also indicates that Example 3 in which the oxidizing agent aqueous solution was dropped on the harvested flowers increased the apigenin concentration in the extract composition as compared with Comparative Example 2 in which the oxidizing agent aqueous solution was dropped on the flowers before harvest. The amount of apigenin in the extract compositions of Examples 1 to 4 was 0.05% by mass or more.

### 2-1. Preparation of Liquid Compositions for Applying Oxidative Stress

### (Example 5)

As the liquid composition for applying an oxidative stress, the same oxidizing agent aqueous solution as that in Example 2 was prepared.

### 2-2. Production Method of Extract Composition

### (Example 5)

An oxidative stress was applied to harvested chamomile using the prepared oxidizing agent aqueous solution to obtain an extract composition of Example 5.

### [Harvest of Asteraceae Plant]

Potted seedlings of German chamomile (Japanese name: kamitsure) were planted in a hydroponic culture system (temperature, 25°C) to grow. As a liquid fertilizer for hydroponic culture, HYPONICA (manufactured by KYOWA CO., LTD.) was diluted 1000 times. After flowering of German chamomile, only the flowers were harvested.

### [Process (1)]

The oxidizing agent aqueous solution was dropped with a dropper on the flowers within one day of harvest. The amount of the oxidizing agent aqueous solution dropped thereon was three times (2.4 g) the wet weight (unit: 0.8 g) of the flowers. The flowers were blended with the oxidizing agent aqueous solution and left to stand at 25°C for three days under indoor conditions.

### [Process (2)]

The flowers after the process (1) were immersed in squalane in an amount 3 times (0.45 g) the dry weight (0.15 g) of the flowers after the process (1). The immersion temperature was 60°C, and the immersion time was 6 hours.

Thereafter, the squalane (immersion solution), in which the flowers were immersed, was filtered with a filter (disposable filter manufactured by ADVANTEC, pore diameter, 0.45 µm) to obtain an extract composition.

### (Comparative Example 3)

An extract composition of Comparative Example 3 was obtained in the same manner as in Example 5 except that the oxidizing agent aqueous solution was not dropped on the harvested flowers.

### (Comparative Example 4)

An extract composition of Comparative Example 4 was obtained in the same manner as in Example 5 except that the oxidizing agent aqueous solution was dropped on the flowers before harvest.

### 2-3. Evaluation

### [Evaluation of Spiro-Ether Content]

The spiro-ether content in the obtained extract composition was measured using high performance liquid chromatography (HPLC) under conditions below. Table 2 shows the results of the spiro-ether concentration, the spiro-ether concentration relative to the case of not applying an oxidative stress (Comparative Example 3), and the spiro-ether concentration relative to the case of applying an oxidative stress before harvest (Comparative Example 4).

### <Measurement Conditions>

Column: InertSustain C18 (manufactured by GL Sciences Inc.)
Eluant: formic acid 0.1% (solution A) and acetonitrile (solution B)
Gradient conditions: gradient from 95 (solution A) : 5 (solution B) to 10 (solution A): 90 (solution B)
Flow rate: 0.5 mL/min
Column temperature: 40°C
Detector: CAD

**[Table 2]**

| Table 2 | Liquid composition for applying oxidative stress | | Timing to apply oxidative stress to German chamomile (flowers) | Extract composition | | |
|---|---|---|---|---|---|---|
| | Oxidative stress applying agent | | | Spiro-ether concentration (ppm) | Relative to the case of not applying oxidative stress | Relative to the case of applying oxidative stress before harvest |
| | Kind | Concentration | | | | |
| Ex. 5 | Hydrogen peroxide | 0.1 mM | After harvest | 987 | 2.2 | 2.4 |
| Comp. Ex. 3 | - | - | - | 444 | 1.0 | 1.1 |
| Comp. Ex. 4 | Hydrogen peroxide | 0.1 mM | Before harvest | 408 | 0.9 | 1.0 |

| | | | | | | |
|---|---|---|---|---|---|---|
| * Ex.: Example, Comp. Ex.: Comparative Example | | | | | | |

Table 2 above indicates that Example 5 in which the application of an oxidative stress with use of the oxidizing agent aqueous solution was performed increased the spiro-ether concentration in the extract composition as compared with Comparative Example 3 in which the application of an oxidative stress with use of the oxidizing agent aqueous solution was not performed. Table 2 also indicates that Example 5 in which the oxidizing agent aqueous solution was dropped on the harvested flowers increased the spiro-ether concentration in the extract composition as compared with Comparative Example 4 in which the oxidizing agent aqueous solution was dropped on the flowers before harvest.

### 3-1. Preparation of Liquid Compositions for Applying Oxidative Stress

### (Examples 6 to 8)

As the liquid composition for applying an oxidative stress, the same oxidizing agent aqueous solution as that in Example 2 was prepared.

### 3-2. Production Method of Extract Composition

### (Examples 6 to 8)

An oxidative stress was applied to harvested herbaceous plant using the prepared oxidizing agent aqueous solution to obtain extract compositions of Examples 6 to 8.

### [Harvest of Herbaceous Plants]

Harvest of Umbelliferae Angelica keiskei leaves: Example 6
Harvest of Lamiaceae peppermint leaves: Example 7
Harvest of Guttiferae Saint John's wort leaves: Example 8

### [Process (1)]

The oxidizing agent aqueous solution was dropped with a dropper on the leaves within one day of harvest. The amount of the oxidizing agent aqueous solution dropped thereon was three times (2.4 g) the wet weight (unit: 0.8 g) of the flowers. The flowers were blended with the oxidizing agent aqueous solution and left to stand at 25°C for three days under indoor conditions.

### [Process (2)]

The leaves after the process (1) were immersed in a 1,3-butylene glycol aqueous solution (concentration, 60% by mass) in an amount 20 times (3.0 g) the dry weight (0.15 g) of the leaves after the process (1). The immersion temperature was 40°C, and the immersion time was one day.

Thereafter, the 1,3-butylene glycol aqueous solution (immersion solution), in which the leaves were immersed, was filtered with a filter (disposable filter manufactured by ADVANTEC, pore diameter, 0.45 pm) to obtain an extract composition.
Extract composition of Angelica keiskei: Example 6
Extract composition of peppermint: Example 7
Extract of Saint John's wort: Example 8

### (Comparative Examples 5, 7 and 9)

Extract compositions of Comparative Examples 5, 7 and 9 were obtained in the same manner as in Examples 6 to 8 except that the oxidizing agent aqueous solution was not dropped on the harvested leaves.

### (Comparative Examples 6, 8 and 10)

Extract compositions of Comparative Examples 6, 8 and 10 were obtained in the same manner as in Examples 6 to 8 except that the oxidizing agent aqueous solution was dropped on the leaves before harvest.

### 3-3. Evaluation

### [Evaluation of Content of Extract Components]

The chlorogenic acid and luteolin concentrations in the extract composition of Angelica keiskei, the rosmarinic acid and luteolin concentrations in the extract composition of peppermint, and the rutin concentration in the extract of Saint John's wort were measured under conditions below. Tables 3 to 5 show the results of the concentration, the concentration relative to the case of not applying an oxidative stress (Comparative Examples 5, 7 and 9), and the concentration relative to the case of applying an oxidative stress before harvest (Comparative Examples 6, 8 and 10).

### <Measurement Conditions>

Column: InertSustain C 18 (manufactured by GL Sciences Inc.)
Eluant: formic acid 0.1% (solution A) and acetonitrile (solution B)
Gradient conditions: gradient from 95 (solution A) : 5 (solution B) to 10 (solution A): 90 (solution B)
Flow rate: 0.5 mL/min
Column temperature: 40°C
Detector: CAD

**[Table 3]**

| Table 3 | Liquid composition for applying oxidative stress | | Timing to apply oxidative stress to Angelica keiskei (leaves) | Extract composition | | | | | |
|---|---|---|---|---|---|---|---|---|---|
| | Oxidative stress applying agent | | | Chlorogenic acid cone. (ppm) | Relative to the case of not applying oxidative stress | Relative to the case of applying oxidative stress before harvest | Luteolin conc. (ppm) | Relative to the case of not applying oxidative stress | Relative to the case of applying oxidative stress before harvest |
| | Kind | Conc. | | | | | | | |
| Ex. 6 | Hydrogen peroxide | 0.1 mM | After harvest | 553 | 11.8 | 9.2 | 67 | 3.9 | 1.6 |
| Comp. Ex. 5 | - | - | - | 47 | 1.0 | 0.8 | 17 | 1.0 | 0.4 |
| Comp. Ex. 6 | Hydrogen peroxide | 0.1 mM | Before harvest | 60 | 1.3 | 0.8 | 42 | 2.5 | 1.0 |

**[Table 4]**

| Table 4 | Liquid composition for applying oxidative stress | | Timing to apply oxidative stress to peppermint (leaves) | Extract composition | | | | | |
|---|---|---|---|---|---|---|---|---|---|
| | Oxidative stress applying agent | | | Rosmarinic acid cone. (ppm) | Relative to the case of not applying oxidative stress | Relative to the case of applying oxidative stress before harvest | Luteolin conc. (ppm) | Relative to the case of not applying oxidative stress | Relative to the case of applying oxidative stress before harvest |
| | Kind | Conc. | | | | | | | |
| Ex. 7 | Hydrogen peroxide | 0.1 mM | After harvest | 1358 | 1.6 | 1.7 | 31 | 7.8 | 15.5 |
| Comp. Ex. 7 | - | - | - | 854 | 1.0 | 1.1 | 4 | 1.0 | 2.0 |
| Comp. Ex. 8 | Hydrogen peroxide | 0.1 mM | Before harvest | 781 | 0.9 | 1.0 | 2 | 0.5 | 1.0 |

**[Table 5]**

| Table 5 | Liquid composition for applying oxidative stress | | Timing to apply oxidative stress to Saint John's wort (leaves) | Extract composition | | |
|---|---|---|---|---|---|---|
| | Oxidative stress applying agent | | | Rutin conc. (ppm) | Relative to the case of not applying oxidative stress | Relative to the case of applying oxidative stress before harvest |
| | Kind | Conc. | | | | |
| Ex. 8 | Hydrogen peroxide | 0.1 mM | After harvest | 838 | 1.5 | 1.3 |
| Comp. Ex. 9 | - | - | - | 565 | 1.0 | 0.9 |
| Comp. Ex. 10 | Hydrogen peroxide | 0.1 mM | Before harvest | 635 | 1.1 | 1.0 |

| | | | | | | |
|---|---|---|---|---|---|---|
| * Ex.: Example, Comp. Ex.: Comparative Example, conc.: concentration | | | | | | |

Tables 3 to 5 above indicate that Examples 6 to 8 in which the application of an oxidative stress with use of the oxidizing agent aqueous solution was performed increased the concentration of the components in the extract compositions as compared with Comparative Examples 5, 7 and 9 in which the application of an oxidative stress with use of the oxidizing agent aqueous solution was not performed. Tables 3 to 5 also indicate that Examples 6 to 8 in which the oxidizing agent aqueous solution was dropped on the harvested flowers increased the concentration of the components in the extract compositions as compared with Comparative Examples 6, 8 and 10 in which the oxidizing agent aqueous solution was dropped on the flowers before harvest.

### 4-1. Preparation of Liquid Compositions for Applying Oxidative Stress

### (Examples 9 and 10)

As the liquid compositions for applying an oxidative stress of Example 9 and Example 10, an oxidizing agent aqueous solution containing nitric acid as the oxidizing agent and an oxidizing agent aqueous solution containing hypochlorous acid as the oxidizing agent were prepared, respectively.

### 4-2. Production Method of Extract Composition

### [Harvest of Asteraceae Plant]

The same Roman chamomile flowers as those in Examples 1 to 4 were used.

### [Process (1)]

The process (1) was performed in the same manner as in Examples 1 to 4.

### [Process (2)]

The process (2) was performed in the same manner as in Examples 1 to 4 to obtain extract compositions.

### (Comparative Examples 11 and 12)

Extract compositions of Comparative Examples 11 and 12 were obtained in the same manner as in Examples 9 and 10 except that the oxidizing agent aqueous solution was dropped on the flowers before harvest.

### 4-3. Evaluation

### [Evaluation of Apigenin Content]

The apigenin content in the obtained extract composition was measured under the same conditions as those in Examples 1 to 4. Tables 6 and 7 show the results of the apigenin concentration, the apigenin concentration relative to the case of not applying an oxidative stress (Comparative Example 1), and the apigenin concentration relative to the case of applying an oxidative stress before harvest (Comparative Examples 11 and 12).

**[Table 6]**

| Table 6 | Liquid composition for applying oxidative stress | | Timing to apply oxidative stress to Roman chamomile (flowers) | Extract composition | | |
|---|---|---|---|---|---|---|
| | Oxidative stress applying agent | | | Apigenin conc. (ppm) | Relative to the case of not applying oxidative stress | Relative to the case of applying oxidative stress before harvest |
| | Kind | Conc. | | | | |
| Ex. 9 | Nitric acid | | After harvest | 836 | 1.5 | 1.2 |
| Comp. Ex. 1 | - | - | - | 572 | 1.0 | 0.8 |
| Comp. Ex. 11 | Nitric acid | | Before harvest | 709 | 1.2 | 1.0 |

**[Table 7]**

| Table 7 | Liquid composition for applying oxidative stress | | Timing to apply oxidative stress to Roman chamomile (flowers) | Extract composition | | |
|---|---|---|---|---|---|---|
| | Oxidative stress applying agent | | | Apigenin conc. (ppm) | Relative to the case of not applying oxidative stress | Relative to the case of applying oxidative stress before harvest |
| | Kind | Conc. | | | | |
| Ex. 10 | Hypochlorous acid | | After harvest | 859 | 1.5 | 1.2 |
| Comp. Ex. 1 | - | - | - | 572 | 1.0 | 0.8 |
| Comp. Ex. 12 | Hypochlorous acid | | Before harvest | 709 | 1.2 | 1.0 |

| | | | | | | |
|---|---|---|---|---|---|---|
| * Ex.: Example, Comp. Ex.: Comparative Example, conc.: concentration | | | | | | |

Tables 6 and 7 above indicate that Examples 9 and 10 in which the application of an oxidative stress with use of the oxidizing agent aqueous solution was performed increased the apigenin concentration in the extract compositions as compared with Comparative Example 1 in which the application of an oxidative stress with use of the oxidizing agent aqueous solution was not performed. Tables 6 and 7 also indicate that Examples 9 and 10 in which the oxidizing agent aqueous solution was dropped on the harvested flowers increased the apigenin concentration in the extract compositions as compared with Comparative Examples 11 and 12 in which the oxidizing agent aqueous solution was dropped on the flowers before harvest.

### Industrial Applicability

The present disclosure provides a method for producing an extract composition by which, for example, polyphenols such as apigenin and/or essential oil components such as spiro-ether can be extracted efficiently.

## Claims

1. A method for producing an extract composition, comprising processes (1) and (2) below:
(1) applying an oxidative stress to a harvested herbaceous plant and preserving it; and
(2) obtaining an extract from the herbaceous plant that has undergone the process (1).

2. A method for producing an extract composition, comprising processes (1) and (2) below:
(1) applying an oxidative stress to a harvested apigenin-containing plant and preserving it; and
(2) obtaining an extract from the apigenin-containing plant that has undergone the process (1).

3. The method according to claim 1 or 2, wherein the herbaceous plant in the process (1) is a part or an entire of the plant.

4. The method according to any of claims 1 to 3, wherein the application of an oxidative stress is an application of an oxidative stress with use of a liquid composition comprising an oxidizing agent.

5. The method according to claim 4, wherein the liquid composition comprises a spreading agent in addition to the oxidizing agent.

6. The method according to any of claims 1 and 3 to 5, wherein the herbaceous plant comprises at least one of a polyphenol or an essential oil component.

7. The method according to any of claims 1 to 6, wherein the plant in the process (1) is a plant belonging to the family Asteraceae, Umbelliferae, Lamiaceae, or Guttiferae.

8. The method according to any of claims 1 to 7, wherein the plant in the process (1) is German chamomile, Angelica keiskei, peppermint, Saint John's wort, or Roman chamomile.

9. A method for producing an external product or food, comprising a process of producing an extract composition by the method according to any of claims 1 to 8.

10. An extract composition that is produced by the method according to any of claims 1 to 8,
wherein the extract composition comprises apigenin in an amount of 0.001% by mass or more.

11. An external product or food comprising the extract composition according to claim 10 or a purified product thereof.
